# EUROPEAN PATENT SPECIFICATION

(11) **EP 1 093 366 B1**
(45) Date of publication and mention of the grant of the patent: **13.10.2004**
(21) Application number: 99930482.7
(22) Date of filing: 18.06.1999
(51) Int. Cl.: A61K 31/195, A61K 31/52, A61P 31/22

(54) **PHARMACEUTICAL COMPOSITION CONTAINING GABA ANALOGS AND AN ANTIVIRAL AGENT TO TREAT SHINGLES**
PHARMAZEUTISCHE ZUSAMMENSETZUNGEN ENTHALTEND GABA-ANALOGEN UND EIN ANTIVIRALES MITTEL ZUR BEHANDLUNG VON HERPES ZOSTER
COMPOSITION PHARMACEUTIQUE RENFERMANT DES ANALOGUES D'ACIDE GAMMA-AMINOBUTYRIQUE (GABA) ET UN AGENT ANTIVIRAL AUX FINS DU TRAITEMENT DE L'HERPES ZOSTER

(30) Priority: 09.07.1998 US 92171 P
(43) Date of publication of application: 25.04.2001
(73) Proprietor: Warner-Lambert Company LLC, Morris Plains, New Jersey 07950 (US)
(72) Inventor: MAGNUS, Leslie, Livingston, NJ 07039 (US); SEGAL, Catherine, A., Chester, NJ 07930-2707 (US)
(74) Representative: Mansmann, Ivo
(86) International application number: PCT/US1999/013947
(87) International publication number: WO 2000/002592

(56) References cited:
- WO-A-95/09632
- WO-A-97/33859
- WO-A-98/03167
- GB-A- 2 282 759
- ROSENBERG J M ET AL: "The effect of gabapentin on neuropathic pain" THE CLINICAL JOURNAL OF PAIN, vol. 13, no. 3, September 1997 (1997-09), pages 251-255, XP002101738
- R.W. JOHNSON: "Current and future management of herpes zoster" ANTIVIRAL CHEMISTRY AND CHEMOTHERAPY, vol. 8, no. S. 1, 1997, pages 19-29, XP002117663
- SATTERTHWAITE J R: "ACUTE HERPES ZOSTER: DIAGNOSIS AND TREATMENT" PAIN MANAGEMENT, vol. 3, no. 1, 1 January 1990 (1990-01-01), pages 17-28, XP000574537 ISSN: 0896-9132
- NIKKELS A F ET AL: "RECOGNITION AND TREATMENT OF SHINGLES" DRUGS, vol. 48, no. 4, 1 October 1994 (1994-10-01), pages 528-548, XP000567542 ISSN: 0012-6667

## Description

### 1. Field Of The Invention

The present invention relates to the use of analogs of glutamic acid and gamma-aminobutyric acid (GABA) in combination with an antiviral agent, for the treatment of shingles.

### 2. Description of Related Art

The GABA analogs used in the present invention are known agents useful in antiseizure therapy for central nervous system disorders such as epilepsy, Huntington's chorea, cerebral ischemia, Parkinson's disease, tardive dyskinesia, and spasticity. It has also been suggested that the compounds can be used as antidepressants, anxiolytics, and antipsychotics. See WO 92/09560 (United States Serial Number 618,692 filed November 27, 1990) and WP 93/23383 (United States Serial Number 886,080 filed May 20, 1992).

WO 97/33858 teaches that compounds related to gabapentin are useful or treating epilespy, faintness attacks, hypokinesia, cranial disorders, neurodegenerative disorders, depression, anxiety, panic, pain, and neuropathological disorders. WO 97/33858 does not specify what forms of pain are treated.

Additionally, the GABA analogs compounds of the present invention are known for treatment of neuropathic pain. For example, see Rosner H; Rubin L; Kestenbaum A., Gabapentin adjunctive therapy in neuropathic pain states. Clin J Pain, 1996 Mar, 12:1, 56-8; Segal AZ; Rordorf G., Gabapentin as a novel treatment for postherpetic neuralgia. Neurology, 1996 Apr, 46:4, 1175-6; Wetzel CH; Connelly JF., Use of gabapentin in pain management. Ann Pharmacother, 1997 Sep, 31:9, 1082-3; Zapp JJ., Postpoliomyelitis pain treated with gabapentin [letter]. Am Fam Physician, 1996 Jun, 53:8, 2442, 2445; Cheville A, et al., Neuropathic pain in radiation myelopathy:a case report. Program book, American Pain Society (14th Annual Scientific Meeting). Abstract #95823, p. A-115; Sist T; Filadora V; Miner M; Lema M., Gabapentin for idiopathic trigeminal neuralgia: report of two cases. Neurology, 1997 May, 48:5, 1467; Waldman SD, Tutorial 28: Evaluation and Treatment of Trigeminal Neuralgia. Pain Digest (1997) 7:21-24; Mellick LB; Mellick GA., Successful treatment of reflex sympathetic dystrophy with gabapentin [letter]. Am J Emerg Med, 1995 Jan, 13:1, 96; Mellick GA; Seng MI., The use of gabapentin in the treatment of reflex sympathetic dystrophy and a phobic disorder. Am J Pain Manage 1995; 5:7-9; Mellick GA; Mellicy LB; Mellick LB., Gabapentin in the management of reflex sympathetic dystrophy [letter]. J Pain Symptom Manage, 1995 May, 10:4, 265-6; Mellick GA; Mellick LB., Reflex sympathetic dystrophy treated with gabapentin. Arch Phys Med Rehabil, 1997 Jan, 78:1, 98-105 and Mackin GA., Medical and pharmacologic management of upper extremity neuropathic pain syndromes. J Hand Ther, 1997 Apr-Jun, 10:2, 96-109.

U.S. Patent No. 5,589,180 teaches a plaster composition for treating pain from herpes zoster or post perpetic neuralgia comprising an adhesive containing 2-10% by weight lidocaine, at least one of propylene glycol and glycerin as a cosolvent and a covering.

Antiviral compounds are known to treat herpes. Thes compounds include acyclovir, famciclovir, valacylovir, peniclovir and mixtures thereof. These antiviral compounds interfere with the enxyme thymidine kinase that is needed to for the replication of the herpes virus.

### SUMMARY OF THE INVENTION

This invention provides the use of an effective amount of a GABA analog and an antiviral agent for the preparation of a pharmaceutical composition for treating shingles. A preferred embodiment utilizes a cyclic amino acid compound of Formula I 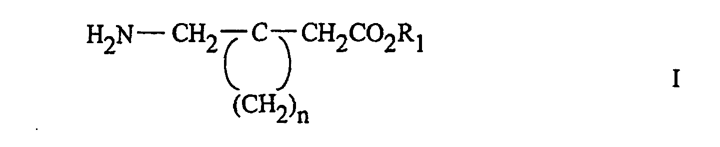 wherein R₁ is hydrogen or lower alkyl and n is an integer of from 4 to 6, and the pharmaceutically acceptable salts thereof. An especially preferred embodiment utilizes a compound of Formula I where R₁ is hydrogen and n is 4, which compound is 1-(aminomethyl)-cyclohexane acetic acid, known generically as gabapentin.

In another embodiment, the invention includes treating shingles with a compound of Formula II and an antiviral agent.
Formula II 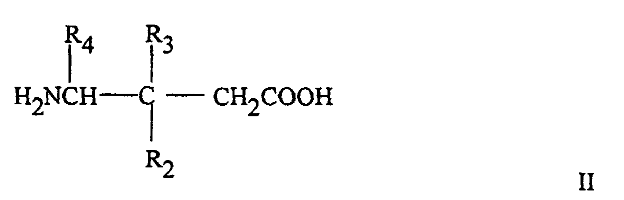 wherein R₂ is a straight or branched alkyl of from 1 to 6 carbon atoms, phenyl, or cycloalkyl having from 3 to 6 carbon atoms; R₃ is hydrogen or methyl; and R₄ is hydrogen, methyl, or carboxyl; or an individual enantiomeric isomer thereof; or a pharmaceutically acceptable salt thereof, in unit dosage form, to a mammal in need of said treatment.

Preferred compounds of the invention are those wherein R₄ and R₃ are hydrogen, and R₂ is -(CH₂)₀₋₂-i C₄H₉ as an (R), (S), or (R,S) isomer.

The more preferred compounds of Formula II invention are (S)-3-(aminomethyl)-5-methylhexanoic acid and 3-aminomethyl-5-methyl-hexanoic acid, now known generically as pregabalin.

### DETAILED DESCRIPTION OF THE PREFERRED EMBODIMENTS

This invention utilizes any GABA analog. A GABA analog is any compound derived from or based upon gamma-aminobutyric acid. The compounds are readily available, either commercially, or by synthetic methodology well-known to those skilled in the art of organic chemistry. The preferred GABA analogs to be utilized in this invention are cyclic amino acids of Formula I. These are described in U.S. Patent 4,024,175. Another preferred embodiment utilizes the GABA analogs of Formula II, and these are described in U.S. Patent 5,563,175.

All that is required to practice this invention is to use a GABA analog in an amount that is effective to treat shingles. Such amounts will generally be from about 1 to about 300 mg per kg of subject body weight. Typical doses will be from about 10 to about 5000 mg per day for an adult subject of normal weight. It is expected that common doses that might be administered could be from 100 mg three times a day up to 600 mg four times a day. Commercially available capsules of 100 mg, 300 mg, and 400 mg of gabapentin can be administered. Alternate forms include liquids and film-coated tablets.

If a compound of Formula II, such as pregabalin is used, the dosage level is one sixth that of gabapentin. The dosage range for pregabalin is from about 0.15 mg to about 50 mg per kg per day of subject body weight. Typical dosages for pregabalin will be from about 1.6 mg to about 840 mg per day with individual dosages ranging from abut 0.15 mg to about 65 mg per dose.

The compounds used in the present invention may form pharmaceutically acceptable salts with both organic and inorganic acids or bases. For example, the acid addition salts of the basic compounds are prepared either by dissolving the free base in aqueous or aqueous alcohol solution or other suitable solvents containing the appropriate acid and isolating the salt by evaporating the solution. Examples of pharmaceutically acceptable salts are hydrochlorides, hydrobromides, hydrosulfates, etc. as well as sodium, potassium, and magnesium, etc. salts.

The compounds of the Formula II can contain one or several asymmetric carbon atoms. The invention includes the individual diastereomers or enantiomers, and the mixtures thereof. The individual diastereomers or enantiomers may be prepared or isolated by methods already well-known in the art.

Pharmaceutical compositions of the compound of the present invention or its salts are produced by formulating the active compound in dosage unit form with a pharmaceutical carrier. Some examples of dosage unit forms are tablets, capsules, pills, powders, aqueous and nonaqueous oral solutions and suspensions, and parenteral solutions packaged in containers containing either one or some larger number of dosage units and capable of being subdivided into individual doses. Some examples of suitable pharmaceutical carriers, including pharmaceutical diluents, are gelatin capsules; sugars such as lactose and sucrose; starches such as corn starch and potato starch, cellulose derivatives such as sodium carboxymethyl cellulose, ethyl cellulose, methyl cellulose, and cellulose acetate phthalate; gelatin; talc; stearic acid; magnesium stearate; vegetable oils such as peanut oil, cottonseed oil, sesame oil, olive oil, corn oil, and oil of theobroma; propylene glycol, glycerin; sorbitol; polyethylene glycol; water; agar; alginic acid; isotonic saline, and phosphate buffer solutions; as well as other compatible substances normally used in pharmaceutical formulations. The compositions of the invention can also contain other components such as coloring agents, flavoring agents, and/or preservatives. These materials, if present, are usually used in relatively small amounts. The compositions can, if desired, also contain other therapeutic agents.

The percentage of the active ingredients in the foregoing compositions can be varied within wide limits, but for practical purposes it is preferably present in a concentration of at least 10% in a solid composition and at least 2% in a primary liquid composition. The most satisfactory compositions are those in which a much higher proportion of the active ingredient is present.

Routes of administration of the subject compound or its salts are oral or parenteral. For example, a useful intravenous dose is between 5 and 50 mg and a useful oral dosage is between 20 and 800 mg. The dosage is within the dosing range used in treatment of pain or as would be with the needs of the patient as described by the physician.

A unit dosage form of the GABA analog to be used in this invention may also comprise other compounds useful in the treatment of pain.

The advantages of using the compounds of Formula I and II, especially gabapentin and pregabalin, in the instant invention include the relatively nontoxic nature of the compounds, the ease of preparation, the fact that the compounds are well-tolerated, and the ease of IV administration of the drugs. Gabapentin has few interactions with major classes of drugs since it is not metabolized in the liver, but rather excreted unchanged from the body. Further, the drugs are not metabolized in the body. The subjects treated with the compositions of the present invention are mammals, including humans.

The antiviral compositions used in the present invention reduce the viral load thereby reducing the number of days of suffering. GABA analogs have no direct impact on the viral load. The GABA analogs work to diminish the pain signals begin transmitted from the peripheral nerves to the brain. The combination of actions improve control and pain relief during a shingles infection.

## Claims

1. Use of
(a) an analgesically effective amount of a GABA analog; and
(b) an effective amount of a anti-viral agent for the preparation of a pharmaceutical composition for treating shingles.

2. The use according to claim 1, wherein the GABA analog is the compound according to Formula I: 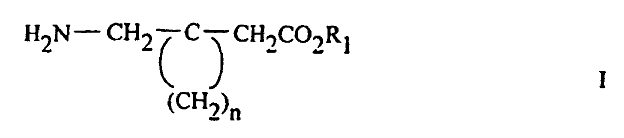 wherein R₁ is hydrogen or lower alkyl and n is an integer of from 4 to 6, and the pharmaceutically acceptable salts thereof.

3. The use according to claim 2, wherein Formula I comprises gabapentin.

4. The use according to claim 1, wherein the anti-viral agent is selected from the group consisting of acyclovir, famciclovir, valacylovir, peniclovir and mixtures thereof.

5. The use according to claim 2, comprising from about 10 mg to about 400 mg of Formula I.

6. The use according to claim 3, comprising from about 10 mg to about 400 mg of gabapentin.

7. The use according to claim 3, comprising from about 10 mg to about 400 mg of gabapentin and from about 60 mg to about 200 mg of anti-viral agent.

8. The use according to claim 1, wherein the GABA analog is a compound according to Formula II: 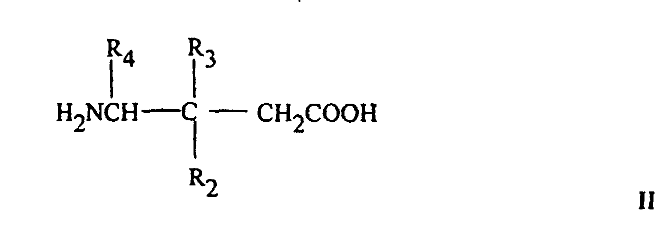 wherein R₂ is a straight or branched alkyl of from 1 to 6 carbon atoms, phenyl, or cycloalkyl having from 3 to 6 carbon atoms; R₃ is hydrogen or methyl; and R₄ is hydrogen, methyl, or carboxyl.

9. The use according to claim 8, wherein Formula II comprises pregabalin.

10. The use according to claim 8, comprising from about 0.15 mg to about 65 mg of Formula II.

11. The use according to claim 9, comprising from about 0.15 mg to about 65 mg of pregabalin.

12. A pharmaceutical composition comprising:
(a) an analgesically effective amount of a GABA analog; and
(b) an effective amount of a anti-viral agent and a
pharmaceutically acceptable carrier.

13. The composition according to claim 12, wherein the GABA analog is a compound according to Formula I: 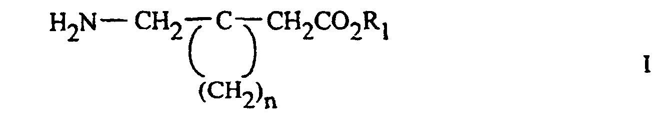 wherein R₁ is hydrogen or lower alkyl and n is an integer of from 4 to 6, and the pharmaceutically acceptable salts thereof.

14. The composition according to claim 13, wherein Formula I comprises gabapentin.

15. The composition according to claim 13, comprising from about 10 mg to about 400 mg of Formula I.

16. The composition according to claim 14, comprising from about 10 mg to about 400 mg of gabapentin.

17. The composition according to claim 12, wherein the GABA analog is a compound according to Formula II: 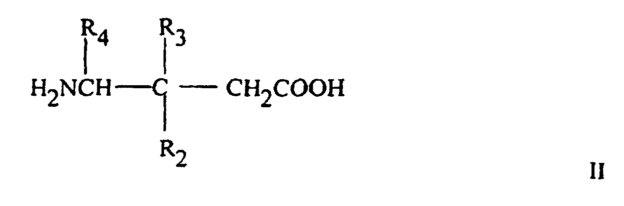 wherein R₂ is a straight or branched alkyl of from 1 to 6 carbon atoms, phenyl, or cycloalkyl having from 3 to 6 carbon atoms; R₃ is hydrogen or methyl; and R₄ is hydrogen, methyl, or carboxyl.

18. The composition according to claim 17, wherein Formula II comprises pregabalin.

19. The composition according to claim 17, comprising from about 0.15 mg to about 65 mg of Formula II.

20. The composition according to claim 19, comprising from about 0.15 mg to about 65 mg of pregabalin.

## Patentansprüche

1. Verwendung
(a) einer analgetisch wirksamen Menge eines GABA-Analogons und
(b) einer wirksamen Menge eines antiviralen Mittels zur Herstellung einer pharmazeutischen Zusammensetzung zur Behandlung von Gürtelrose.

2. Verwendung nach Anspruch 1, wobei das GABA-Analogon die Verbindung gemäß der Formel I: 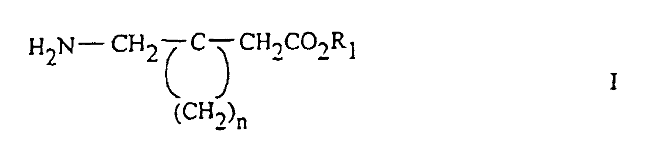 worin R₁ Wasserstoff oder Niederalkyl bedeutet und n eine ganze Zahl von 4 bis 6 bedeutet, und die pharmazeutisch akzeptablen Salze derselben sind.

3. Verwendung nach Anspruch 2, wobei die Formel I Gabapentin umfasst.

4. Verwendung nach Anspruch 1, wobei das antivirale Mittel aus der aus Acyclovir, Famciclovir, Valacylovir, Peniclovir und Gemischen derselben bestehenden Gruppe ausgewählt ist.

5. Verwendung nach Anspruch 2, die etwa 10 mg bis etwa 400 mg der Formel I umfasst.

6. Verwendung nach Anspruch 3, die etwa 10 mg bis etwa 400 mg Gabapentin umfasst.

7. Verwendung nach Anspruch 3, die etwa 10 mg bis etwa 400 mg Gabapentin und etwa 60 mg bis etwa 200 mg eines antiviralen Mittels umfasst.

8. Verwendung nach Anspruch 1, wobei das GABA-Analogon eine Verbindung gemäß der Formel II ist: 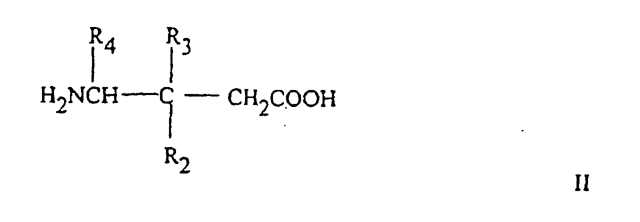 worin R₂ ein gerades oder verzweigtes Alkyl mit 1 bis 6 Kohlenstoffatomen, Phenyl oder Cycloalkyl mit 3 bis 6 Kohlenstoffatomen bedeutet; R₃ Wasserstoff oder Methyl bedeutet; und R₄ Wasserstoff, Methyl oder Carboxyl bedeutet.

9. Verwendung nach Anspruch 8, wobei die Formel II Pregabalin umfasst.

10. Verwendung nach Anspruch 8, die etwa 0,15 mg bis etwa 65 mg der Formel II umfasst.

11. Verwendung nach Anspruch 9, die etwa 0,15 mg bis etwa 65 mg Pregabalin umfasst.

12. Pharmazeutische Zusammensetzung, die umfasst:
(a) eine analgetisch wirksame Menge eines GABA-Analogons und
(b) eine wirksame Menge eines antiviralen Mittels und einen pharmazeutisch akzeptablen Träger.

13. Zusammensetzung nach Anspruch 12, wobei das GABA-Analogon eine Verbindung der Formel I 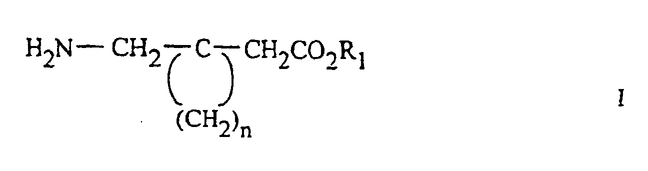 ist
worin R₁ Wasserstoff oder Niederalkyl bedeutet und n eine ganze Zahl von 4 bis 6 bedeutet, und die pharmazeutisch akzeptablen Salze desselben.

14. Zusammensetzung nach Anspruch 13, wobei die Formel I Gabapentin umfasst.

15. Zusammensetzung nach Anspruch 13, die etwa 10 mg bis etwa 400 mg der Formel I umfasst.

16. Zusammensetzung nach Anspruch 14, die etwa 10 mg bis etwa 400 mg Gabapentin umfasst.

17. Zusammensetzung nach Anspruch 12, wobei das GABA-Analogon eine Verbindung gemäß der Formel II 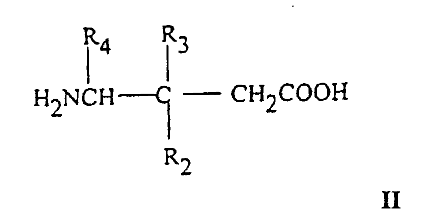 ist
worin R₂ ein gerades oder verzweigtes Alkyl mit 1 bis 6 Kohlenstoffatomen, Phenyl oder Cycloalkyl mit 3 bis 6 Kohlenstoffatomen bedeutet, R₃ für Wasserstoff oder Methyl steht und R₄ Wasserstoff, Methyl oder Carboxyl bedeutet.

18. Zusammensetzung nach Anspruch 17, wobei die Formel II Pregabalin umfasst.

19. Zusammensetzung nach Anspruch 17, die etwa 0,15 mg bis etwa 65 mg der Formel II umfasst.

20. Zusammensetzung nach Anspruch 19, die etwa 0,15 mg bis etwa 65 mg Pregabalin umfasst.

## Revendications

1. Utilisation de:
(a) une quantité analgésiquement efficace d'un analogue de GABA et
(b) une quantité efficace d'un agent antiviral
pour la préparation d'une composition pharmaceutique pour le traitement du zona.

2. Utilisation selon la revendication 1, dans laquelle l'analogue du GABA est le composé de formule I: 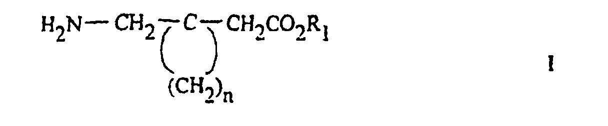 dans laquelle R₁ représente l'hydrogène ou un alkyle inférieur et n représente un entier compris entre 4 et 6, ainsi que les sels pharmaceutiques acceptables de ce composé.

3. Utilisation selon la revendication 2, dans laquelle la formule I comprend la gabapentine.

4. Utilisation selon la revendication 1, dans laquelle l'agent antiviral est choisi dans le groupe constitué de l'acyclovir, du famciclovir, du valacylovir, du péniclovir et des mélanges de ceux-ci.

5. Utilisation selon la revendication 2, qui comprend d'environ 10 mg à environ 400 mg d'un composé de formule I.

6. Utilisation selon la revendication 3, qui comprend d'environ 10 mg à environ 400 mg de gabapentine.

7. Utilisation selon la revendication 3, qui comprend d'environ 10 mg à environ 400 mg de gabapentine et d'environ 60 mg à environ 200 mg d'agent antiviral.

8. Utilisation selon la revendication 1, dans laquelle l'analogue du GABA est un composé selon la formule II: 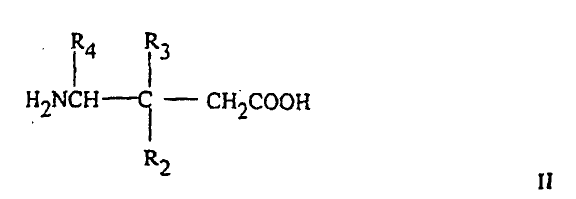 dans laquelle R₂ représente un alkyle linéaire ou ramifié qui compte de 1 à 6 atomes de carbone, le phényle ou un cycloalkyle qui compte de 3 à 6 atomes de carbone; R₃ représente l'hydrogène ou le méthyle et R₄ représente l'hydrogène, le méthyle ou le carboxyle.

9. Utilisation selon la revendication 8, dans laquelle la formule II comprend la prégabaline.

10. Utilisation selon la revendication 8, qui comprend d'environ 0,15 mg à environ 65 mg d'un composé de formule II.

11. Utilisation selon la revendication 9, qui comprend d'environ 0,15 mg à environ 65 mg de prégabaline.

12. Composition pharmaceutique qui comprend:
(a) une quantité analgésiquement efficace d'un analogue du GABA et
(b) une quantité efficace d'un agent antiviral et un support pharmaceutiquement acceptable.

13. Composition selon la revendication 12, dans laquelle un analogue du GABA est un composé selon la formule I: 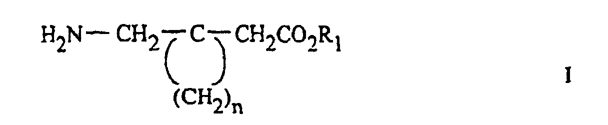 dans laquelle R₁ représente l'hydrogène ou un alkyle inférieur et n un entier compris entre 4 et 6, et les sels pharmaceutiquement acceptables de ce composé.

14. Composition selon la revendication 13, dans laquelle la formule I comprend la gabapentine.

15. Composition selon la revendication 13, qui comprend d'environ 10 mg à environ 400 mg d'un composé de formule I.

16. Composition selon la revendication 14, qui comprend d'environ 10 mg à environ 400 mg de gabapentine.

17. Composition selon la revendication 12, dans laquelle l'analogue du GABA est un composé de formule II: 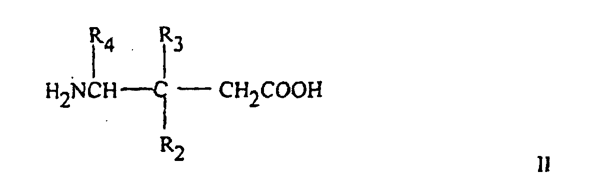 dans laquelle R₂ représente un alkyle linéaire ou ramifié qui compte de 1 à 6 atomes de carbone, le phényle ou un cycloalkyle qui compte de 3 à 6 atomes de carbone; R₃ représente l'hydrogène ou le méthyle et R₄ représente l'hydrogène, le méthyle ou le carboxyle.

18. Composition selon la revendication 17, dans laquelle la formule II comprend la prégabaline.

19. Composition selon la revendication 17, qui comprend d'environ 0,15 mg à environ 65 mg d'un composé de formule II.

20. Composition selon la revendication 19, qui comprend d'environ 0,15 mg à environ 65 mg de prégabaline.
